# EUROPEAN PATENT SPECIFICATION

(11) **EP 4 012 050 B1**
(45) Date of publication and mention of the grant of the patent: **29.11.2023**
(21) Application number: 20927649.2
(22) Date of filing: 19.06.2020
(51) Int. Cl.: C12Q 1/70, C12Q 1/6851, C12N 15/11, C12R 1/93, C12Q 1/6883

(54) **COMPOSITION, KIT AND METHOD FOR DETECTING AND TYPING VIRUSES CAUSING RESPIRATORY TRACT INFECTION AND APPLICATION OF COMPOSITION, KIT AND METHOD**
ZUSAMMENSETZUNG, KIT UND VERFAHREN ZUR DETEKTION UND TYPISIERUNG VON VIREN, DIE EINE INFEKTION DER ATEMWEGE VERURSACHEN, SOWIE ANWENDUNG DER ZUSAMMENSETZUNG, KIT UND VERFAHREN
COMPOSITION, KIT ET PROCÉDÉ DE DÉTECTION ET DE TYPAGE DE VIRUS PROVOQUANT UNE INFECTION DES VOIES RESPIRATOIRES ET APPLICATION DE LA COMPOSITION, DU KIT ET DU PROCÉDÉ

(30) Priority: 23.03.2020 CN 202010205841
(43) Date of publication of application: 15.06.2022
(73) Proprietor: Sansure Biotech Inc., Changsha, Hunan 410205 (CN)
(72) Inventor: DAI, Lizhong, Changsha, Hunan 410205 (CN); TAN, Deyong, Changsha, Hunan 410205 (CN); DENG, Zhongping, Changsha, Hunan 410205 (CN); LIU, Jia, Changsha, Hunan 410205 (CN); SUN, Qingzhi, Changsha, Hunan 410205 (CN); CHENG, Xing, Changsha, Hunan 410205 (CN); REN, Xiaomei, Changsha, Hunan 410205 (CN); CHEN, Xiaoliang, Changsha, Hunan 410205 (CN); MAO, Junjie, Changsha, Hunan 410205 (CN)
(74) Representative: Novagraaf Technologies
(86) International application number: PCT/CN2020/096940
(87) International publication number: WO 2021/189681

(56) References cited:
- CN-A- 102 181 579
- CN-A- 110 343 784
- CN-A- 111 074 011
- Tianlong Technology: "Tianlong Technology Launches Four Nucleic Acid Test Solutions to Facilitate Differential Diagnosis of New Coronavirus", China Biologics Network , 27 February 2020 (2020-02-27), pages 1-3, XP009532044, CN Retrieved from the Internet: URL:https://www.bio-equip.com/news.asp?ID= 453082151
- Victor M Corman, Olfert Landt, Marco Kaiser, Richard Molenkamp, Adam Meijer, Daniel Kw Chu, Tobias Bleicker, Sebastian Brünink, Ju: "Detection of 2019 novel coronavirus (2019-nCoV) by real-time RT-PCR", Eurosurveillance, Centre Europeen pour la Surveillance Epidemiologique du SIDA, FR, vol. 25, no. 3, 23 January 2020 (2020-01-23), XP055695049, FR ISSN: 1560-7917, DOI: 10.2807/1560-7917.ES.2020.25.3.2000045

## Description

### TECHNICAL FIELD

The present invention pertains to the field of molecular biological detection, specifically, to the field of detection of viruses that cause respiratory tract infections. More specifically, the present invention is capable of simultaneous detection and typing of novel coronavirus 2019-nCoV, influenza A virus, and influenza B virus.

### BACKGROUND

Respiratory diseases caused by respiratory tract infections are common diseases, and the causative pathogens mainly include viruses, bacteria, mycoplasmas, chlamydiae, etc. Among them, common viral pathogens include influenza A viruses and influenza B viruses, which are also the main pathogens of seasonal influenza; and another type of virus that causes respiratory tract infections is coronaviruses.

The envelope of influenza viruses has two very important glycoproteins: hemagglutinin (HA) and neuraminidase (NA). Generally, there are 500 hemagglutinin spikes and 100 neuraminidase spikes distributed on the surface of the influenza viruses, which serve as the basis for influenza A virus typing according to the antigenicity of HA and NA in the influenza viruses. Influenza A viruses can infect humans, mammals, and birds. H1N1, H2N2, and H3N2 subtypes mainly infect humans, and other subtypes mainly infect birds, pigs, horses, and aquatic mammals. There are 12 subtypes of avian influenza viruses that can infect humans, including H5N1, H7N1, H7N2, H7N4, H7N9, H9N2, H10N8, etc. Influenza B viruses are mainly prevalent in the population, and has no subtype classification. The three branches are the Li line, the Yamagata line, and the Victoria line, and their epidemic scales are smaller than that of influenza A viruses. The genetic material of influenza viruses is a negative-sense single-stranded RNA (ss-RNA), which binds to a nucleoprotein and winds into a ribonucleoprotein complex present in a form with an extremely high density. In addition to the ribonucleoprotein complex, there is also an RNA polymerase responsible for RNA transcription. The RNA of influenza viruses is mainly composed of eight segments. The first, second, and third segments encode an RNA polymerase, the fourth segment encodes a hemagglutinin; the fifth segment encodes a nucleoprotein; the sixth segment encodes a neuraminidase; the seventh segment encodes a matrix protein; and the eighth segment encodes a nonstructural protein that can function to splice an RNA.

So far, there are seven kinds of coronaviruses infecting humans, among which coronavirus 229E, coronavirus NL63, coronavirus OC43, and coronavirus HKU1 are common ones, which cause mild conditions after infecting the human respiratory tract and usually do not induce severe illnesses. However, novel coronavirus 2019-nCoV, coronavirus MERSr-CoV, and coronavirus SARSr-CoV infections will cause very serious infection symptoms in patients, and are highly likely to develop into severe illnesses.

The novel coronavirus is a new type of coronavirus belonging to the genus β, which is named "novel coronavirus (2019-nCoV)" by the World Health Organization. 2019-nCoV has an envelope, and the particles are round or oval, often pleomorphic, with a diameter of 60-140 nm. The genetic characteristics thereof are significantly different from those of SARSr-CoV and MERSr-CoV. According to the latest research results of researchers, 2019-nCoV is very similar to RaTG13, a bat coronavirus strain previously isolated from Chinese horseshoe bats, with an overall genome similarity of 96.2%. In addition, studies have shown that 2019-nCoV is 85% or more homologous to bat SARS-like coronaviruses (bat-SL-CoVZC45 and bat-SL-CoVZXC21).

The pathogens of respiratory tract infections are extremely diverse. One clinical manifestation may be caused by multiple pathogens, and one pathogen may also cause multiple clinical manifestations, which bring great difficulties to early clinical diagnosis.

For most viruses that cause respiratory tract infections, early treatment will produce better therapeutic effects. For example, once a patient with influenza shows symptoms, he/she should start taking oseltamivir as soon as possible, ideally within 48 hours of symptom onset; when being used for prevention, oseltamivir should also be taken within 48 hours of exposure to a patient with flu. Early diagnosis and early treatment are keys to improving the cure rate and reducing the fatality rate of influenza patients.

For the novel coronavirus, in addition to early detection and early treatment, it is also necessary to control the source of infection in time, so as to stop virus pandemics and outbreaks.

Therefore, distinguishing whether a respiratory tract infection is caused by a novel coronavirus or caused by an influenza A or B virus so as to provide guidance for subsequent prevention and control, treatment, and adoption of targeted measures is the top priority of respiratory disease prevention and control.

Therefore, there is a need in the art for a related product capable of detecting and distinguishing a novel coronavirus, an influenza A virus, and an influenza B virus, whereas document CN110343784 discloses amplification based detection of influenza viruses, including A and B as well as coronavirus.

### SUMMARY

In view of this, provided in the present invention is a composition capable of detecting and typing viruses that cause a respiratory tract infection. The composition simultaneously includes:
a novel coronavirus 2019-nCoV forward primer as shown in SEQ ID NO: 1, a novel coronavirus 2019-nCoV reverse primer as shown in SEQ ID NO: 2, and a novel coronavirus 2019-nCoV probe as shown in SEQ ID NO: 3; and at least one of the following two groups:
a first group: an influenza A virus forward primer as shown in SEQ ID NO: 7, an influenza A virus reverse primer as shown in SEQ ID NO: 8, and an influenza A virus probe as shown in SEQ ID NO: 9; and
a second group: an influenza B virus forward primer as shown in SEQ ID NO: 10, an influenza B virus reverse primer as shown in SEQ ID NO: 11, and an influenza B virus probe as shown in SEQ ID NO: 12,
where fluorescent groups of the probes are different from each other and do not interfere with each other.

Herein, the description "different from each other and do not interfere with each other" means that the fluorescent groups used by the respective probes in the composition are different, and will not affect the detection of each other, i.e., different channels can be used for detection. For example, FAM, HEX, ROX, and CY5 can be used. The absorbance values of these fluorescent groups are not close, and different detection channels can be selected, and therefore, the fluorescent groups will not interfere with each other.

In one preferred embodiment, the composition further includes: a novel coronavirus 2019-nCoV forward primer as shown in SEQ ID NO: 4, a novel coronavirus 2019-nCoV reverse primer as shown in SEQ ID NO: 5, and a novel coronavirus 2019-nCoV probe as shown in SEQ ID NO: 6.

By including the additional SEQ ID NOs: 4-6, the composition of the present invention can further increase the accuracy of novel coronavirus detection, and avoid false negatives such as misses to the greatest extent.

In one specific embodiment, the composition includes: the novel coronavirus 2019-nCoV forward primer as shown in SEQ ID NO: 1, the novel coronavirus 2019-nCoV reverse primer as shown in SEQ ID NO: 2, and the novel coronavirus 2019-nCoV probe as shown in SEQ ID NO: 3; and
the influenza A virus forward primer as shown in SEQ ID NO: 7, the influenza A virus reverse primer as shown in SEQ ID NO: 8, and the influenza A virus probe as shown in SEQ ID NO: 9.

The above embodiment can be used when there is no actual clinical need to detect an influenza B virus, and a simultaneous detection of a novel coronavirus and an influenza A virus is needed, thereby reducing costs.

In one specific embodiment, the composition includes the novel coronavirus 2019-nCoV forward primer as shown in SEQ ID NO: 1, the novel coronavirus 2019-nCoV reverse primer as shown in SEQ ID NO: 2, and the novel coronavirus 2019-nCoV probe as shown in SEQ ID NO: 3; and
the influenza B virus forward primer as shown in SEQ ID NO: 10, the influenza B virus reverse primer as shown in SEQ ID NO: 11, and the influenza B virus probe as shown in SEQ ID NO: 12.

The above embodiment can be used when there is no actual clinical need to detect the influenza A virus, and a simultaneous detection of the novel coronavirus and the influenza B virus is needed, thereby reducing costs.

In one specific embodiment, the composition includes the novel coronavirus 2019-nCoV forward primer as shown in SEQ ID NO: 1, the novel coronavirus 2019-nCoV reverse primer as shown in SEQ ID NO: 2, and the novel coronavirus 2019-nCoV probe as shown in SEQ ID NO: 3;
the influenza A virus forward primer as shown in SEQ ID NO: 7, the influenza A virus reverse primer as shown in SEQ ID NO: 8, and the influenza A virus probe as shown in SEQ ID NO: 9; and
the influenza B virus forward primer as shown in SEQ ID NO: 10, the influenza B virus reverse primer as shown in SEQ ID NO: 11, and the influenza B virus probe as shown in SEQ ID NO: 12.

The above embodiment is used when there is a need to detect the three viruses, and can more comprehensively detect the virus that causes the infection, and a set of primers is used for each virus, thereby reducing costs.

In one specific embodiment, the composition includes the novel coronavirus 2019-nCoV forward primer as shown in SEQ ID NO: 1, the novel coronavirus 2019-nCoV reverse primer as shown in SEQ ID NO: 2, and the novel coronavirus 2019-nCoV probe as shown in SEQ ID NO: 3;
the novel coronavirus 2019-nCoV forward primer as shown in SEQ ID NO: 4, the novel coronavirus 2019-nCoV reverse primer as shown in SEQ ID NO: 5, and the novel coronavirus 2019-nCoV probe as shown in SEQ ID NO: 6;
the influenza A virus forward primer as shown in SEQ ID NO: 7, the influenza A virus reverse primer as shown in SEQ ID NO: 8, and the influenza A virus probe as shown in SEQ ID NO: 9; and
the influenza B virus forward primer as shown in SEQ ID NO: 10, the influenza B virus reverse primer as shown in SEQ ID NO: 11, and the influenza B virus probe as shown in SEQ ID NO: 12.

The above embodiment is used when there is a need to detect the three viruses and high detection accuracy is required, and can obtain high detection accuracy and avoid false negatives.

The virus that causes the respiratory tract infection includes: the novel coronavirus 2019-nCoV, the influenza A virus, and the influenza B virus.

Further, the composition includes: an internal standard forward primer, internal standard reverse primer, and internal standard probe for monitoring.

In one specific embodiment, the composition further includes: the internal standard forward primer as shown in SEQ ID NO: 13, the internal standard reverse primer as shown in SEQ ID NO: 14, and the internal standard probe as shown in SEQ ID NO: 15.

In the present invention, the fluorescent reporter group may be selected from FAM, HEX, ROX, VIC, CY5, 5-TAMRA, TET, CY3, and JOE, but is not limited thereto.

In one specific embodiment, the fluorescent reporter groups of the novel coronavirus 2019-nCoV probes as shown in SEQ ID NOs: 3 and 6 are FAM; the fluorescent reporter group of the influenza A virus probe as shown in SEQ ID NO: 9 is HEX; and the fluorescent reporter group of the influenza B virus probe as shown in SEQ ID NO: 12 is ROX.

In one specific embodiment, a fluorescent reporter group of the internal standard probe as shown in SEQ ID NO: 15 is CY5.

Further, the amount of the primer in the composition is 50-150 nM, and the amount of the probe in the composition is 25-75 nM.

In one specific embodiment, the components of the composition of the present invention are in the same package.

Further, the components of the composition of the present invention are present in a mixed form.

In a second aspect, provided in the present invention is a use of the above composition of the present invention in the preparation of a kit for detecting and typing viruses that cause a respiratory tract infection.

The virus that causes the respiratory tract infection includes: the novel coronavirus 2019-nCoV, the influenza A virus, and the influenza B virus.

In a third aspect, provided in the present invention is a kit for detecting and typing viruses that cause a respiratory tract infection, the kit including the above composition of the present invention.

Further, the kit further includes at least one of a nucleic acid release reagent, a dNTP, a reverse transcriptase, a DNA polymerase, a PCR buffer, and Mg²⁺.

Further, the amount of the primer in the composition is 50-150 nM; the amount of the probe in the composition is 25-75 nM; and the amount of the dNTP is 0.2-0.3 mM.

Further, the concentration of the reverse transcriptase is 5 U/µL to 15 U/µL, and for example, the reverse transcriptase may be a murine leukemia reverse transcriptase (MMLV). The concentration of the DNA polymerase is 5 U/µL to 15 U/µL, and for example, the DNA polymerase may be a Taq enzyme.

In one specific embodiment, in addition to the above composition of the present invention, the kit further includes the following components and amounts:

| Component | Volume/concentration in each reaction |
|---|---|
| Mg²⁺ | 4 mM |
| dNTPs (100 mM) | 0.25 mM |
| MMLV (10 U/µL) | 10 U |
| Taq enzyme (5 U/µL) | 5 U |
| PCR buffer (1.5x) | Make up to 50 µL |

In a fourth aspect, a method for detecting and typing viruses that cause a respiratory tract infection is provided, the method including the steps of:
1) releasing a nucleic acid of a testing sample;
2) performing, by using the above composition of the present invention or the above kit of the present invention, a fluorescent quantitative PCR on the nucleic acid obtained in step 1); and
3) obtaining and analyzing the results.

In the present invention, the testing sample may be a throat swab, sputum, a bronchoalveolar lavage fluid, blood, etc., but is not limited thereto.

Further, reaction conditions of the fluorescent quantitative PCR are as follows:
reverse transcription at a temperature of 50°C for 25-35 minutes for 1 cycle; pre-denaturation at a temperature of 94°C for 2-10 minutes for 1 cycle; denaturation at a temperature of 94°C for 10-20 seconds; annealing at a temperature of 60°C for 20-40 seconds for 45-50 cycles.

Using the composition of the present invention can simultaneously detect and type the three viruses that cause respiratory tract infections, so as to identify some patients having fever caused by common seasonal influenza, reduce the waste of medical resources, and reduce the psychological burden on patients and society.

Furthermore, the present invention can quickly diagnose the novel coronavirus 2019-nCoV, provide molecular evidence for early diagnosis, early treatment, and early isolation of the novel coronavirus, and allow adequate preparations for prevention and control of the disease, making it possible to control the source of infection of the highly contagious and hazardous novel coronavirus 2019-nCoV in a timely manner and stop virus pandemics and outbreaks.

The composition of the present invention in combination with a fluorescent probe method enables the use of one tube in one test simultaneously, achieving low costs and high throughput. The present invention enables information of four targets to be given by one tube in a single test, and the operations are simple and convenient, and a result reading process can be completed according to a CT value. The whole detection process is carried out under single-tube closed conditions, avoiding false positives and environmental contamination caused by crossover between samples.

Additionally, using the composition of the present invention is time-efficient, and the total time from acquiring a sample to obtaining a result is about 120 min, greatly improving the detection efficiency.

### BRIEF DESCRIPTION OF THE DRAWINGS

FIG. 1 shows a positive detection result for 2019-nCoV in a FAM channel of the composition of the present invention;
FIG. 2 shows a positive detection result for influenza A virus in a HEX channel of the composition of the present invention;
FIG. 3 shows a positive detection result for influenza B virus in a ROX channel of the composition of the present invention;
FIG. 4 shows a positive detection result for an internal standard in a CY5 channel of the composition of the present invention;
FIGs. 5-8 show reproducibility results of virus detection according to an embodiment of the composition of the present invention;
FIGs. 9-12 show reproducibility results of virus detection according to another embodiment of the composition of the present invention;
FIG. 13 shows a sensitivity result of the composition of the present invention;
FIGs. 14-15 show specificity results of the composition of the present invention;
FIG. 16 shows experimental results of the composition of the present invention and a comparative primer.

### DETAILED DESCRIPTION

The present invention will be described in detail below with reference to specific embodiments and examples, and the advantages and various effects of the present invention will be more clearly presented therefrom. It should be understood by those skilled in the art that these specific embodiments and examples are intended to illustrate the present invention, rather than to limit the present invention.

### Example 1. Primers and probes used in the present invention

The primers and probes used in the present invention are shown in Table 1 below:

**Table 1**

| | |
|---|---|
| Novel coronavirus 2019-nCoV forward primer (ORFlab) (SEQ ID NO: 1) | TGTAGCTTGTCACACCGTTT |
| Novel coronavirus 2019-nCoV reverse primer (ORFlab) (SEQ ID NO: 2) | ATTAGCATAAGCAGTTGTGGCAT |
| Novel coronavirus 2019-nCoV probe (ORFlab) (SEQ ID NO: 3) | ATAGTGAACCGCCACACATGACCA |
| Novel coronavirus 2019-nCoV forward primer (N) (SEQ ID NO: 4) | CTCACTCAACATGGCAAGG |
| Novel coronavirus 2019-nCoV reverse primer (N) (SEQ ID NO: 5) | ACTGAGATCTTTCATTTTACCGTCAC |
| Novel coronavirus 2019-nCoV probe (N) (SEQ ID NO: 6) | CTACTACCGAAGAGCTACCAGACGAA |
| Influenza A virus forward primer (SEQ ID NO: 7) | GTATTACTAAGGGCTTTCACCGA |
| Influenza A virus reverse primer (SEQ ID NO: 8) | ATTCCATTCAAGTCCTCCGATG |
| Influenza A virus probe (SEQ ID NO: 9) | AAAAGAAGGCAATGGTGAGATTTCGC |
| Influenza B virus forward primer (SEQ ID NO: 10) | GAGCTGCCTATGAAGACCTGA |
| Influenza B virus reverse primer (SEQ ID NO: 11) | GCTCCCATTCCTTCTACCTG |
| Influenza B virus probe (SEQ ID NO: 12) | TTTGCTGGAACATGGAAACCCTT |
| Internal standard forward primer (SEQ ID NO: 13) | TTTGGTATCGTGGAAGGACTCA |
| Internal standard reverse primer (SEQ ID NO: 14) | GCCATCACGCCACAGTTTC |
| Internal standard probe (SEQ ID NO: 15) | ACCACAGTCCATGCCATCACTGCC |

Among them, a fluorescent reporter group of the novel coronavirus 2019-nCoV probe was FAM; a fluorescent reporter group of an influenza A virus was HEX; a fluorescent reporter group of an influenza B virus was ROX, a fluorescent reporter group of the internal standard was CY5, and the 3'-end of the probe further had a BHQ1 or BHQ2 quencher group.

### Example 2. Method for detecting and typing viruses that cause a respiratory tract infection

A testing sample of the present invention was a throat swab, sputum, a bronchoalveolar lavage fluid, or blood. A magnetic bead method was used to extract a viral nucleic acid, and the following operations were performed in a sample processing room:
2.1 An appropriate number of 1.5-mL sterilized centrifuge tubes were taken, and labeled as a negative control, a positive control, and a testing sample, respectively. 300 µL of an RNA extraction solution 1 was added to each tube.
2.2 200 µL of the testing sample or the negative control or the positive control was added to each tube. The tube was covered with a cap, and shaken for 10 seconds for through mixing, and subjected to instant centrifugation.
2.3 100 µL of an RNA extraction solution 2-mix was added to each tube (sucked up after through mixing), and the tube was shaken for 10 seconds for through mixing, and left to stand for 10 minutes at room temperature.
2.4 After instant centrifugation, the centrifuge tubes were placed on a separator, and the solution was slowly sucked out after 3 minutes (be careful not to touch a brown substance adhered to the tube wall).
2.5 600 µL of an RNA extraction solution 3 and 200 µL of an RNA extraction solution 4 were added to each tube, and the tube was shaken for 5 seconds for through mixing and subjected to instant centrifugation, and then the centrifuge tube was placed on the separator again.
2.6 After about 3 minutes, the supernatant separated into two layers. A pipette tip was inserted into the bottom of the centrifuge tube, the liquid was slowly sucked up from the bottom completely and discarded. The tube was left to stand for 1 minute and then the residual liquid at the bottom of the tube was completely sucked up and discarded.
2.7 50 µL of PCR-mix was added to each tube, and a pipette tip was used to suck up the PCR-mix to elute the brown residue adhered to the wall of the centrifuge tube. The operation was repeated several times to elute the residue as completely as possible, and then all the eluted brown mixture was transferred to a 0.2-mL PCR reaction tube, and the tube was covered with a cap and transferred to an amplification test zone.

The real-time fluorescent PCR reaction system was configured as follows:

| Component | Volume/concentration in each reaction |
|---|---|
| Mg²⁺ | 4 mM |
| dNTPs (100 mM) | 0.25 mM |
| MMLV (10 U/µL) | 10 U |
| Taq enzyme (5 U/µL) | 5 U |
| Primer | 100 nM |
| Probe | 50 nM |
| PCR buffer (1.5x) | Make up to 50 µL |

The PCR amplification program was set up as follows:

| Step | Temperature | Time | Number of cycles |
|---|---|---|---|
| Reverse transcription | 50°C | 30 minutes | 1 |
| Pre-denaturation | 94°C | 5 minutes | 1 |
| Denaturation | 94°C | 15 seconds | 45 |
| Annealing | 60°C | 30 seconds | |

Result analysis:
1) Target detection signals were FAM, HEX (or VIC), and ROX, and an internal reference detection signal was CY5.
2) Baseline setting: The baseline was generally set to 3-15 cycles, which specifically could be adjusted according to actual situations. The adjustment principle was to select a region where a fluorescence signal is relatively stable before exponential amplification, a starting point (Start) avoiding signal fluctuation in an initial stage of fluorescence collection, and an ending point (End) being less by 1-2 cycles than the Ct of a sample showing earliest exponential amplification. Threshold setting: The setting principle was to make a threshold line just exceed the highest point of a normal negative control.
3) It was first analyzed whether an amplification curve for the internal standard is detected in the CY5 channel and Ct ≤ 39, and if so, it indicated that the current test was effective, and subsequent analysis continued to be carried out:
   A) if a typical S-shaped amplification curve was detected in the FAM channel and Ct < 39, it indicated that the novel coronavirus 2019-nCoV detection result was positive;
   B) if a typical S-type amplification curve was detected in the HEX channel and Ct < 39, it indicated that the influenza A virus detection result was positive;
   C) if a typical S-type amplification curve was detected in the ROX channel and Ct < 39, it indicated that the influenza B detection result was positive.
4) If a Ct for the internal standard was not detected in the CY5 channel or Ct > 39, it indicated that the concentration of the testing sample was excessively low or there was an interfering substance that inhibited the reaction, and the experiment needed to be re-prepared.

### Example 3. Detection results of detecting the positive control by the composition of the present invention

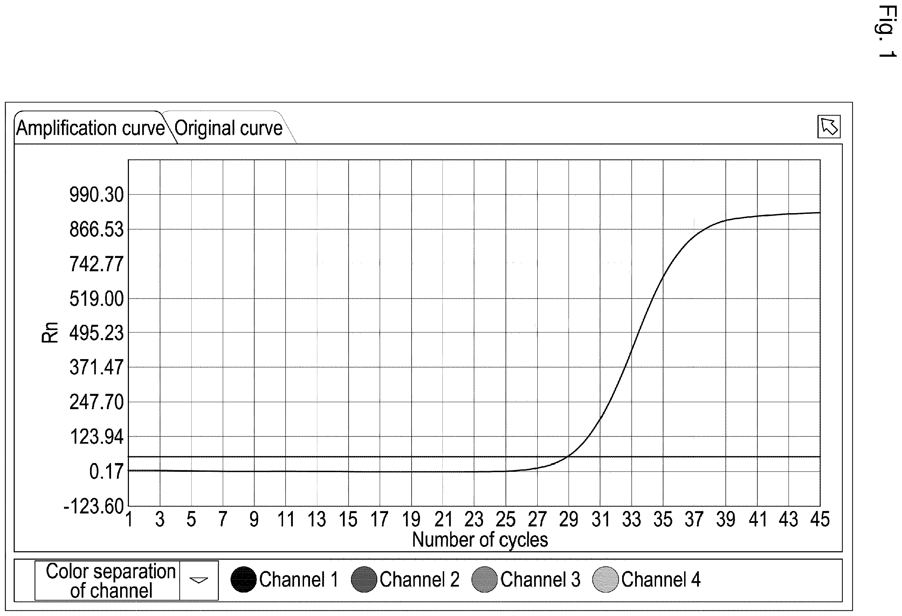

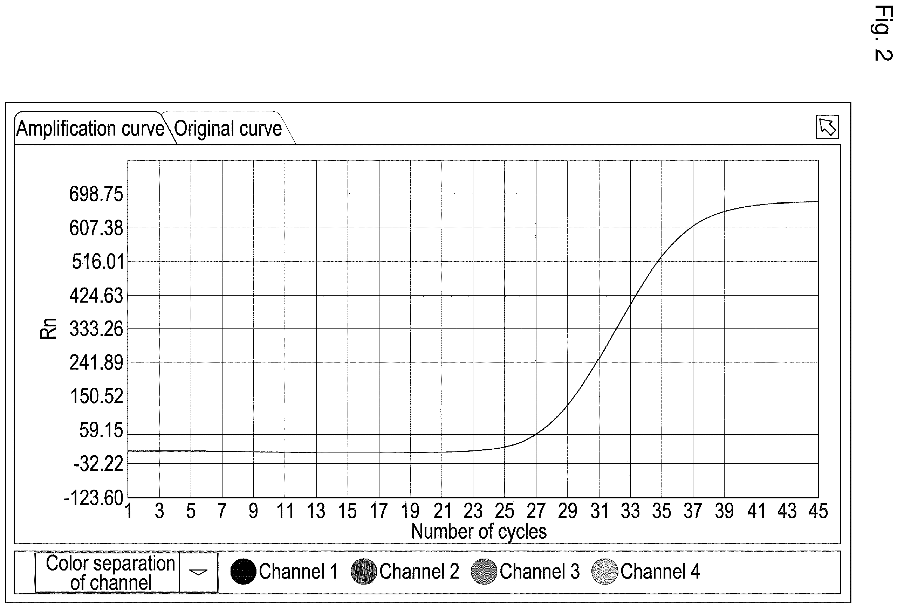

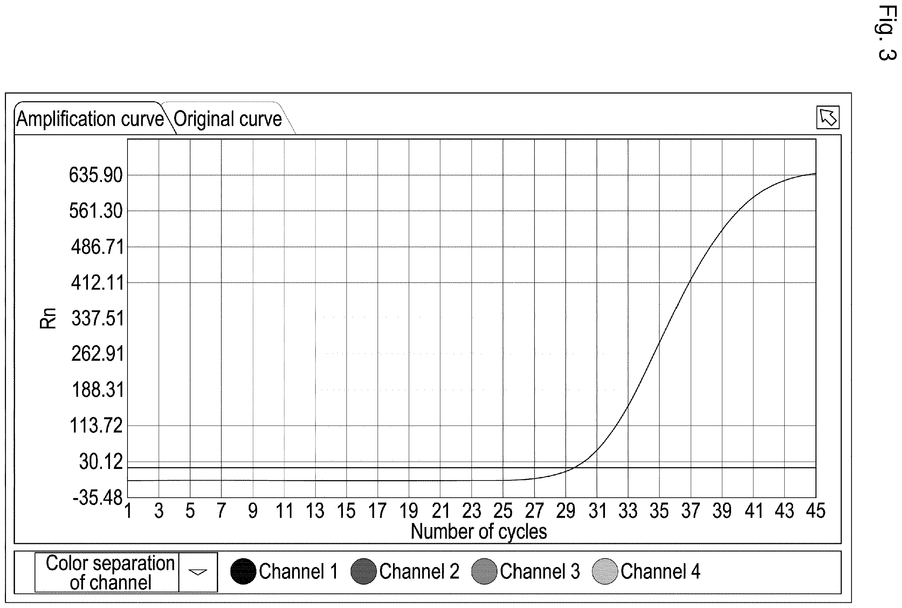

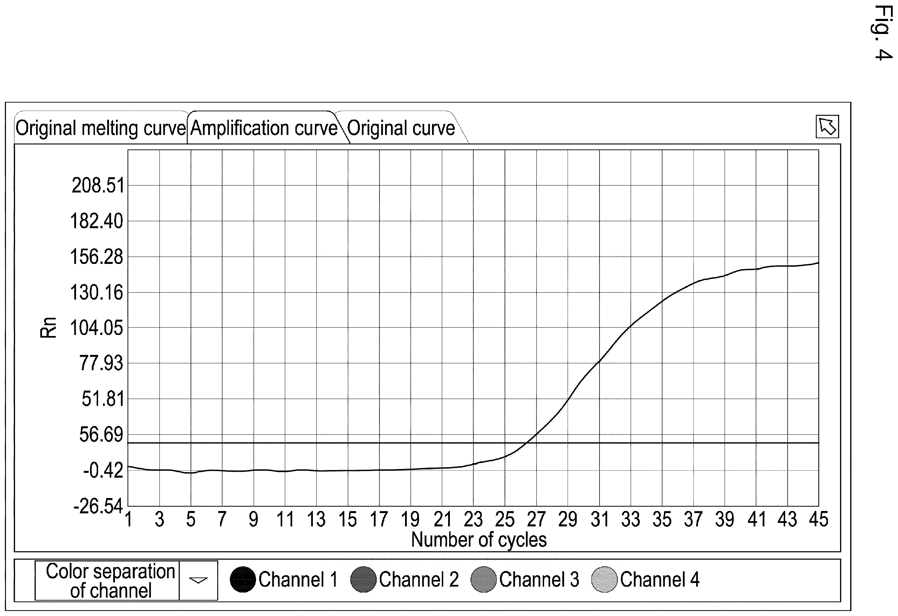

The composition in Table 1 of the present invention was used to detect each target-positive plasmid according to the method described in Example 2, so as to simulate a clinical sample. Multiple PCR tests were conducted on a Hongshi fluorescent quantitative PCR instrument. The results are as shown in FIGs. 1-4. A good amplification curve could be detected in each channel, indicating that the composition of the present invention could detect and type viruses that cause a respiratory tract infection.

### Example 4. Reproducibility results of detecting the positive control by different compositions of the present invention

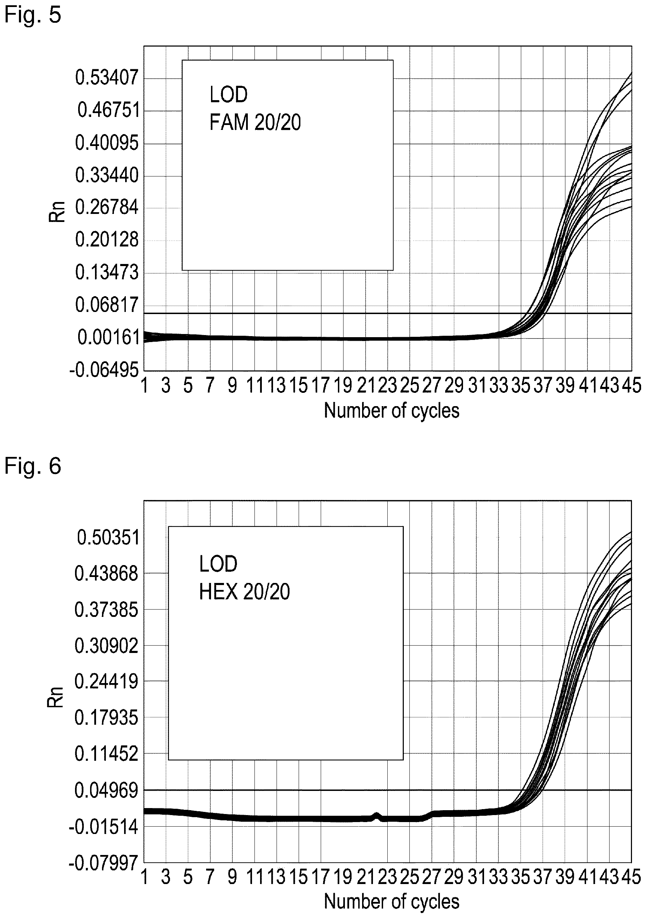

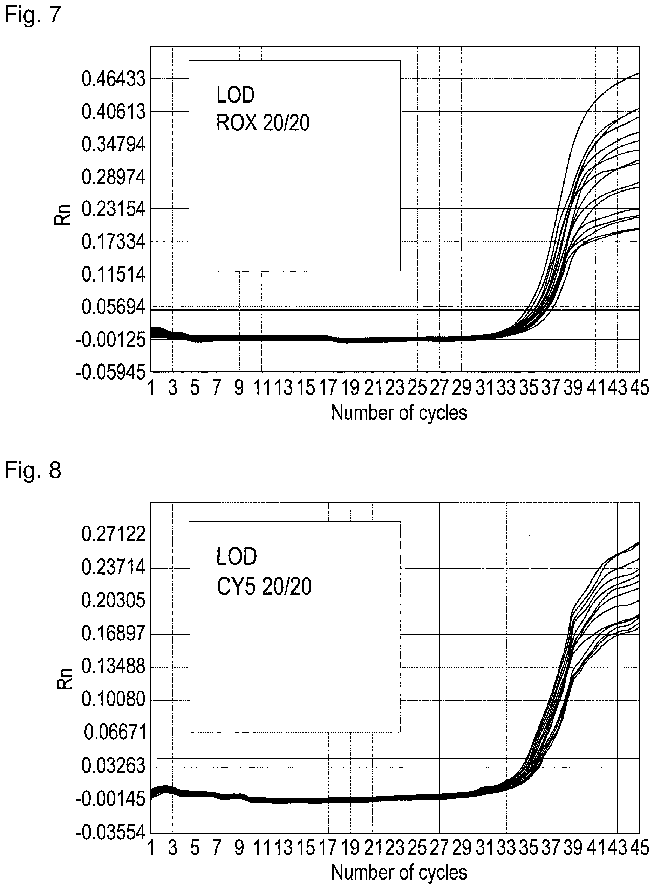

The composition in Table 1 of the present invention was used to detect each target LOD (sensitivity)-positive plasmid according to the method described in Example 2, so as to simulate a clinical sample. Multiple PCR tests were conducted on a Hongshi fluorescent quantitative PCR instrument. The results of this combination are the most desirable, and the virus could be detected in all 20 repetitions, as shown in FIGs. 5-8.

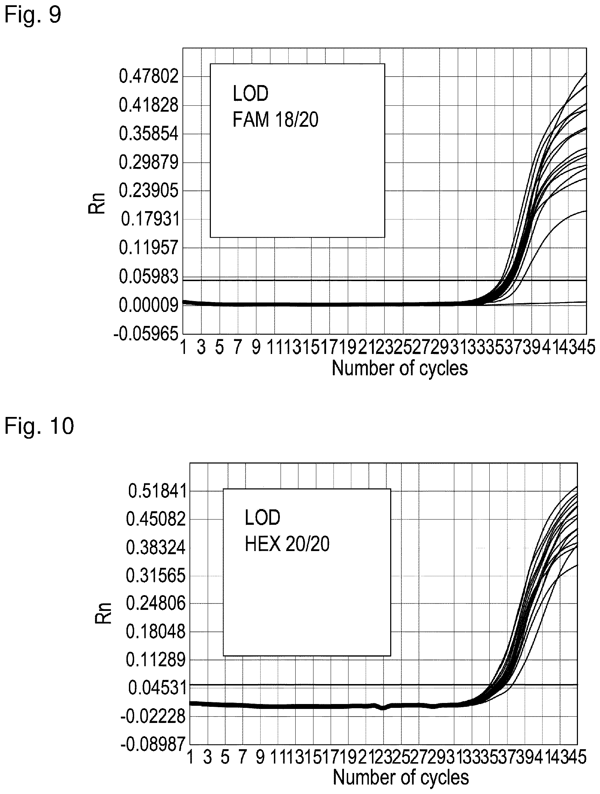

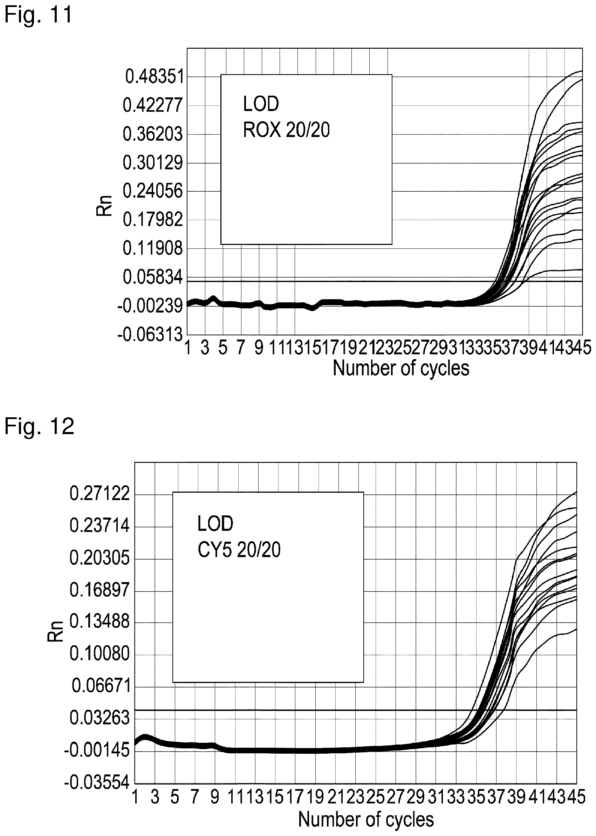

Experiments were carried out using only one set of primers targeting the novel coronavirus (i.e., using only SEQ ID NOs: 1-3) in combination with the primers for the influenza A virus and the influenza B virus. The results of this combination were that the virus could be detected in the HEX, ROX, and CY5 channels in all 20 repetitions, and the virus could be detected in the FAM channel in 18 of 20 repetitions. The results are shown in FIGs. 9-12.

### Example 5. Sensitivity of the composition of the present invention

Experiments were carried out with plasmid templates of different concentrations to test the sensitivity of the composition of the present invention

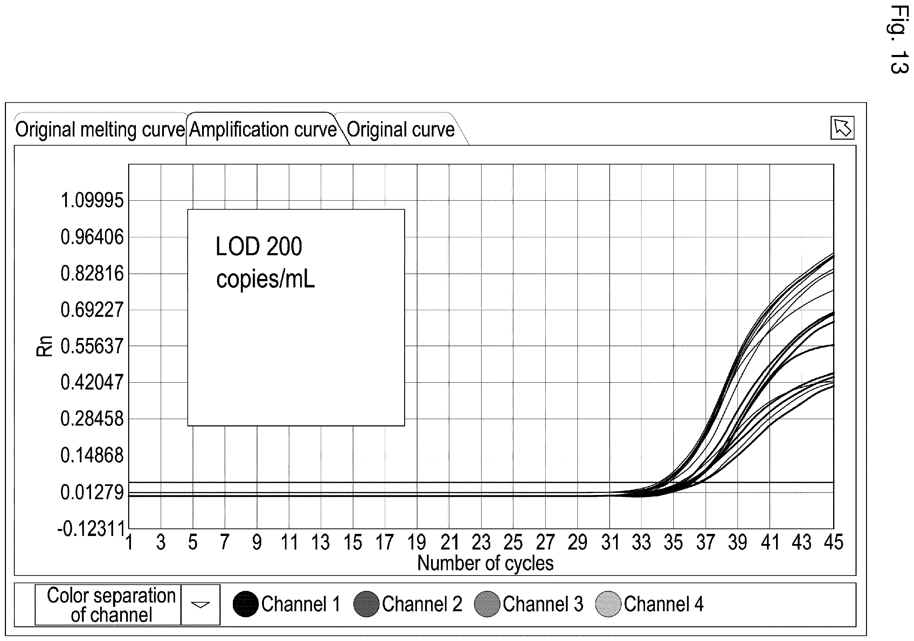

The composition in Table 1 of the present invention was used to detect each target LOD (sensitivity)-positive plasmid according to the method described in Example 2, so as to simulate a clinical sample. Multiple PCR tests were conducted on a Hongshi fluorescent quantitative PCR instrument. The detection sensitivity in each channel was 200 copies/mL. The results show that the composition of the present invention could still detect a virus at a concentration of 200 copies/mL. The results are as shown in FIG. 13, indicating that the sensitivity of the composition of the present invention could reach 200 copies/mL.

### Example 6. Specificity of the composition of the present invention

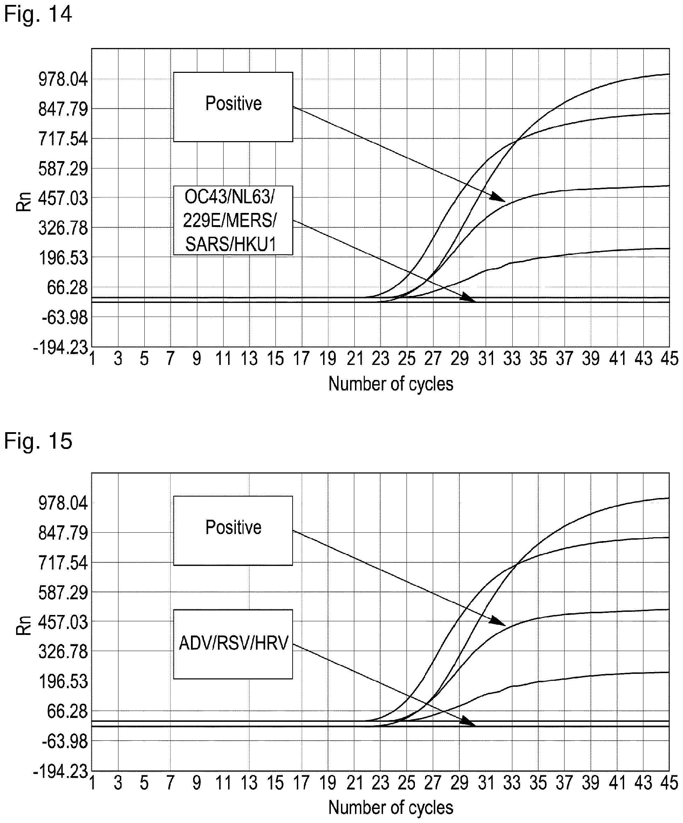

Experiments with some similar viruses showed that the composition of the present invention would not detect other viruses and cause false positives. The results are as shown in FIGs. 14-15, in which the composition of the present invention showed no non-specific amplification to other coronaviruses OC43/OC43/NL63/229E/MERS/SARS/HKU1 as well as common viruses adenovirus ADV, syncytial virus RSV, rhinovirus HRV, indicating that the composition of the present invention has good specificity.

### Comparative Example 1. Other primers and probes designed by the present invention with poor effects

In the process of primer and probe design, the inventor also designed other primers and probes for virus detection, and specific sequences thereof were as follows (all targets are ORF-1ab):
F1 (SEQ ID NO: 16): CCCCAAAATGCTGTTGTTAAAATT
R1 (SEQ ID NO: 17): TTCAAGCCAGATTCATTATGGTATT
P1 (SEQ ID NO: 18):
   FAM-TCCAGCATGTCACAATTCAGAAGTAGGAC-BHQ1
F2 (SEQ ID NO: 19): CCCGCACTCTTGAAACTGCTC
R2 (SEQ ID NO: 20): TAGATTGTTAGTAGCCAAATCAGATGTG
P2 (SEQ ID NO: 21): FAM-CTGTGCGTGTTTTACAGAAGGCCGC-BHQ1
F3 (SEQ ID NO: 22): GTTGCTCGAAATCAAAGACACAGAA
R3 (SEQ ID NO: 23): CTTGTAACCTTGCACTTCTATCACAGT
P3 (SEQ ID NO: 24):
   FAM-TTGCACCTAATATGATGGTAACAAACAATACCT-BHQ1

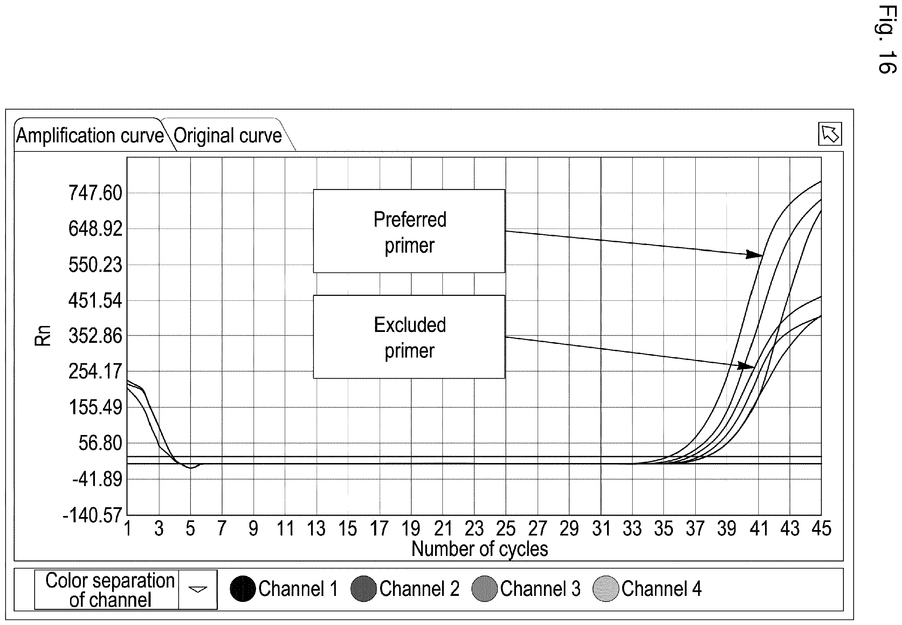

The above composition was used for detection according to the method shown in Example 2. As can be seen from FIG. 16, the primers of the comparative example could not desirably detect positive plasmids, which proved that the primers of the comparative example could not be used to detect viruses that cause a respiratory tract infection.

## Claims

1. A composition capable of detecting and typing viruses that cause a respiratory tract infection, the composition simultaneously comprising:
a novel coronavirus 2019-nCoV forward primer as shown in SEQ ID NO: 1, a novel coronavirus 2019-nCoV reverse primer as shown in SEQ ID NO: 2, and a novel coronavirus 2019-nCoV probe as shown in SEQ ID NO: 3; and at least one of the following two groups:
a first group: an influenza A virus forward primer as shown in SEQ ID NO: 7, an influenza A virus reverse primer as shown in SEQ ID NO: 8, and an influenza A virus probe as shown in SEQ ID NO: 9; and
a second group: an influenza B virus forward primer as shown in SEQ ID NO: 10, an influenza B virus reverse primer as shown in SEQ ID NO: 11, and an influenza B virus probe as shown in SEQ ID NO: 12,
where fluorescent groups of the probes are different from each other and do not interfere with each other.

2. The composition according to claim 1, wherein the composition further comprises: a novel coronavirus 2019-nCoV forward primer as shown in SEQ ID NO: 4, a novel coronavirus 2019-nCoV reverse primer as shown in SEQ ID NO: 5, and a novel coronavirus 2019-nCoV probe as shown in SEQ ID NO: 6.

3. The composition according to claim 2, wherein the composition further comprises an internal standard forward primer, internal standard reverse primer, and internal standard probe for monitoring.

4. The composition according to claim 3, wherein the sequence of the internal standard forward primer is as shown in SEQ ID NO: 13, the sequence of the internal standard reverse primer is as shown in SEQ ID NO: 14, and the sequence of the internal standard probe is as shown in SEQ ID NO: 15.

5. The composition according to any one of claims 1 to 4, wherein components of the composition are in the same package.

6. A use of the composition according to any one of claims 1 to 5 in the preparation of a kit for detecting and typing viruses that cause a respiratory tract infection.

7. A kit for detecting and typing viruses that cause a respiratory tract infection, the kit comprising the composition according to any one of claims 1 to 5.

8. The kit according to claim 7, wherein the kit further comprises at least one of a nucleic acid release reagent, a dNTP, a reverse transcriptase, a DNA polymerase, a PCR buffer, and Mg2+.

9. The kit according to claim 8, wherein the amount of the primer in the composition is 50-150 nM, and the amount of the probe in the composition is 25-75 nM.

10. A method for detecting and typing viruses that cause a respiratory tract infection, the method comprising the steps of:
1) releasing a nucleic acid of a testing sample;
2) performing, by using the composition according to any one of claims 1 to 5, a fluorescent quantitative PCR on the nucleic acid obtained in step 1); and
3) obtaining and analyzing the results.

## Patentansprüche

1. Eine Zusammensetzung, die in der Lage ist, Viren, die eine Infektion der Atemwege verursachen, nachzuweisen und zu typisieren, wobei die Zusammensetzung gleichzeitig umfasst:
einen neuartigen Coronavirus 2019-nCoV-Vorwärtsprimer, wie in SEQ ID NO: 1 gezeigt, einen neuartigen Coronavirus 2019-nCoV-Rückwärtsprimer, wie in SEQ ID NO: 2 gezeigt, und eine neuartige Coronavirus 2019-nCoV-Sonde, wie in SEQ ID NO: 3 gezeigt; und mindestens eine der folgenden zwei Gruppen:
eine erste Gruppe: einen Influenza-A-Virus-Vorwärtsprimer wie in SEQ ID NO: 7 gezeigt, einen Influenza-A-Virus-Rückwärtsprimer wie in SEQ ID NO: 8 gezeigt und eine Influenza-A-Virus-Sonde wie in SEQ ID NO: 9 gezeigt; und
eine zweite Gruppe: einen Influenza-B-Virus-Vorwärtsprimer wie in SEQ ID NO: 10 gezeigt, einen Influenza-B-Virus-Rückwärtsprimer wie in SEQ ID NO: 11 gezeigt und eine Influenza-B-Virus-Sonde wie in SEQ ID NO: 12 gezeigt,
bei denen die Fluoreszenzgruppen der Sonden unterschiedlich sind und sich nicht gegenseitig stören.

2. Zusammensetzung nach Anspruch 1, wobei die Zusammensetzung ferner umfasst: einen neuartigen Coronavirus 2019-nCoV-Vorwärtsprimer wie in SEQ ID NO: 4 gezeigt, einen neuartigen Coronavirus 2019-nCoV-Rückwärtsprimer wie in SEQ ID NO: 5 gezeigt und eine neuartige Coronavirus 2019-nCoV-Sonde wie in SEQ ID NO: 6 gezeigt.

3. Zusammensetzung nach Anspruch 2, wobei die Zusammensetzung ferner einen internen Standard-Vorwärtsprimer, einen internen Standard-Rückwärtsprimer und eine interne Standardsonde zur Überwachung umfasst.

4. Zusammensetzung nach Anspruch 3, wobei die Sequenz des internen Standard-Vorwärtsprimer wie in SEQ ID NO: 13 gezeigt ist, die Sequenz des internen Standard-Rückwärtsprimer wie in SEQ ID NO: 14 gezeigt ist und die Sequenz der Standardsonde wie in SEQ ID NO: 15 gezeigt ist.

5. Zusammensetzung nach einem der Ansprüche 1 bis 4, wobei sich die Bestandteile der Zusammensetzung in derselben Verpackung befinden.

6. Verwendung der Zusammensetzung nach einem der Ansprüche 1 bis 5 zur Herstellung eines Kits zum Nachweis und zur Typisierung von Viren, die für Infektionen der Atemwege verantwortlich sind.

7. Kit zum Nachweis und zur Typisierung von Viren, die eine Infektion der Atemwege verursachen, wobei das Kit die Zusammensetzung nach einem der Ansprüche 1 bis 5 enthält.

8. Kit nach Anspruch 7, wobei der Kit weiterhin mindestens eines der folgenden Elemente umfasst: ein Nukleinsäurefreisetzungsreagenz, ein dNTP, eine reverse Transkriptase, eine DNA-Polymerase, einen PCR-Puffer und Mg²⁺.

9. das Kit nach Anspruch 8, wobei die Menge des Primers in der Zusammensetzung 50-150 nM und die Menge der Sonde in der Zusammensetzung 25-75 nM beträgt.

10. Verfahren zum Nachweis und zur Typisierung von Viren, die eine Infektion der Atemwege verursachen, wobei das Verfahren die folgenden Schritte umfasst:
1) Freisetzung einer Nukleinsäure aus einer Testprobe;
2) Durchführen einer fluoreszierenden quantitativen PCR an der in Schritt 1) erhaltenen Nukleinsäure unter Verwendung der Zusammensetzung nach einem der Ansprüche 1 bis 5; und
3) Gewinnung und Analyse der Ergebnisse.

## Revendications

1. Composition capable de détecter et de typer les virus qui causent une infection des voies respiratoires, la composition comprenant simultanément :
une nouvelle amorce directe du coronavirus 2019-nCoV telle que présentée dans SEQ ID NO : 1, une nouvelle amorce inverse du coronavirus 2019-nCoV telle que présentée dans SEQ ID NO : 2, et une nouvelle sonde du coronavirus 2019-nCoV telle que présentée dans SEQ ID NO : 3 ; et au moins l'un des deux groupes suivants :
un premier groupe : une amorce directe du virus de la grippe A telle que présentée dans SEQ ID NO : 7, une amorce inverse du virus de la grippe A telle que présentée dans SEQ ID NO : 8, et une sonde du virus de la grippe A telle que présentée dans SEQ ID NO : 9 ; et
un deuxième groupe : une amorce directe du virus de la grippe B telle que présentée dans SEQ ID NO : 10, une amorce inverse du virus de la grippe B telle que présentée dans SEQ ID NO : 11, et une sonde du virus de la grippe B telle que présentée dans SEQ ID NO : 12,
où les groupes fluorescents des sondes sont différents les uns des autres et n'interférent pas entre eux.

2. Composition selon la revendication 1, dans laquelle la composition comprend en outre : une nouvelle amorce directe du coronavirus 2019-nCoV telle que présentée dans SEQ ID NO : 4, une nouvelle amorce inverse du coronavirus 2019-nCoV telle que présentée dans SEQ ID NO : 5, et une nouvelle sonde du coronavirus 2019-nCoV telle que présentée dans SEQ ID NO : 6.

3. Composition selon la revendication 2, dans laquelle la composition comprend en outre une amorce directe standard interne, une amorce inverse standard interne et une sonde standard interne pour la surveillance.

4. Composition selon la revendication 3, dans laquelle la séquence de l'amorce directe de standard interne est telle que présentée dans SEQ ID NO : 13, la séquence de l'amorce inverse de standard interne est telle que présentée dans SEQ ID NO : 14, et la séquence de la sonde de standard interne est telle que présentée dans SEQ ID NO : 15.

5. Composition selon l'une des revendications 1 à 4, dans laquelle les composants de la composition sont dans le même emballage.

6. Utilisation de la composition selon l'une des revendications 1 à 5 dans la préparation d'un kit de détection et de typage des virus responsables d'une infection des voies respiratoires.

7. Kit de détection et de typage des virus responsables d'une infection des voies respiratoires, le kit comprenant la composition selon l'une des revendications 1 à 5.

8. Kit selon la revendication 7, dans lequel le kit comprend en outre au moins un des éléments suivants : réactif de libération d'acide nucléique, dNTP, transcriptase inverse, ADN polymérase, tampon PCR, et Mg²⁺.

9. Kit selon la revendication 8, dans lequel la quantité d'amorce dans la composition est de 50-150 nM, et la quantité de sonde dans la composition est de 25-75 nM.

10. Procédé de détection et de typage des virus responsables d'une infection des voies respiratoires, le procédé comprenant les étapes suivantes
1) libération d'un acide nucléique d'un échantillon à tester ;
2) réalisation, en utilisant la composition selon l'une des revendications 1 à 5, d'une PCR quantitative fluorescente sur l'acide nucléique obtenu à l'étape 1) ; et
3) obtention et analyse des résultats.
